# EUROPEAN PATENT APPLICATION

(11) **EP 2 789 686 A1**
(43) Date of publication of application: **15.10.2014**
(21) Application number: 13163354.7
(22) Date of filing: 11.04.2013
(51) Int. Cl.: C12N 5/04, C12N 9/10, C12N 15/82

(54) **Expression of phosphorylated glycoproteins in plants**

(71) Applicant: Greenovation Biotech GmbH, 79111 Freiburg i.Br (DE)
(72) Inventor: Fode, Benjamin, 79211 Denzlingen (DE); Schaaf, Andreas, 79104 Freiburg (DE)
(74) Representative: Sonn & Partner Patentanwälte

(57) **Abstract**

The invention relates to expressing a glycoprotein with a mammalian-type phosphoglycosylation in a plant comprising expressing said glycoprotein in a plant which has been recombinantly modified to express non-plant enzymes capable of modifying the post-translational pathway of the plant to allow the mammalian-type phosphoglycosylation. Recombinant enzymes may be from the group of N-acetylglucosamine-1-phosphotransferase (GNPT), N-acetylglucosamine-1-phosphodiester-alpha-N-acetylglucosaminidase (NAGPA), mannosyl-phosphate-transferase Mnn6p and optional further regulatory proteins.

SEQUENCE LISTING

<110>  greenovation Biotech GmbH

<120>  Expression of phosphorylated glycoproteins in plants

<130>  r63357

<160>  19

<170>  PatentIn version 3.3

<210>  1
<211>  5896
<212>  DNA
<213>  Artificial

<220>
<223>  expression construct

<400>  1
gagctcctcg agccactcct atgccaatag tgactcagtc tagtttcaag aatctcttct      60

tgttattgtc aatagctatt ttagttttca tattatactc ttatataaac atctactctc     120

tttgagtaat ataagaatat attgaagttt tgattgtttg tgattcatat ccttgatact     180

tgtgccaaag tggccaatat atggccaatt tgttgacaaa gttttctact ttttaatcac     240

ctatagacac atataaagtc aaatccacgt ctatgaagct tctaaaaagc tagtatataa     300

gatattatca atatttactt ctatttattt ctatctaact ccttttatct aagtaattcc     360

aagtgttgat tatgtttgta atagcaattg aattgagttt ctaaagcctt gttatgattt     420

taaatttgat taaattaaat tatattttca atataatctt tgaatgttta aaaataaata     480

aataaataaa tcataaggtt gctaatttca ctttgtttat ttaataacat ctaattaact     540

tttcaaatta tttaatcttc taatgctaga aattacaatt tggcttttgc caatgcacaa     600

gtttgacatc ataagcctat cttttctttt cttttttgct tttgaatttt tgcttatttg     660

acaacaacta caatatcgtg tattgtcaga atttcttatt tggctacaat cacggctgtg     720

taacgtgcaa acacaacacc ttcagtctta gtattgacgt ccttgatcat ggcaggacta     780

tagtcaacaa ccgtggtacc cacagcaatg ttcatctcat ctgcaacatg agatgatgcg     840

tcgatggcga gagagtgatg cgctgcgttg ttgtgattgt gtgcaccagc aattaatcat     900

cttctcgccg aaacactggt ttgattcgca ttatcgcaca cagtagcatc tttgaagccg     960

gatctgtgat cggagactct cactgctgaa gataaccgtg cccggacttt gttgccgtag    1020

ggtttattgc agctgaattt gatcattgtg cgctatgttg atgcagccca gcgatgagtg    1080

gtgaggttga tcggcagcga gggcagctga gcgcgttttc gcatgtcaga gctgcctgag    1140

cgtatgcgtt gccgtcaacg atcgagggga atggtgcaga gtgaatttta gggtttctgg    1200

tgctcgtcag aagtttgtgg gcgagtttcc gtcgcgtgtg ggatcgctgg agctgcggtt    1260

tttgttcgct ttggacgtcg ttcatcagaa tggcgttgtt ggaatgcgga gcgcgggttt    1320

ggcttctcgt agctgctgta atgttccttc tcttgcaagc gagattgttt tcagtgcaga 1380

ccaagtatgc agaccggatc gctttggctg tggaagggga gagccgagat caataccatg 1440

ttttgtttga ttcctataga gacaatattg ctggaaagtc ctttcagaat cggctttgtc 1500

tgcccatgcc gattgacgtt gtttacacct gggtgaatgg cacagatctt gaactactga 1560

aggaactaca gcaggtcaga gaacagatgg aggaggagca gaaagcaatg agagaaatcc 1620

ttgggaaaaa cacaacggaa cctactaaga agagtgagaa gcagttagag tgtttgctaa 1680

cacactgcat taaggtgcca atgcttgtcc tggacccagc cctgccagcc aacatcaccc 1740

tgaaggacct gccatctctt tatccttctt ttcattctgc cagtgacatt ttcaatgttg 1800

caaaaccaaa aaacccttct accaatgtct cagttgttgt ttttgacagt actaaggatg 1860

ttgaagatgc ccactctgga ctgcttaaag gaaatagcag acagacagta tggaggggct 1920

acttgacaac agataaagaa gtccctggat tagtgctaat gcaagatttg gctttcctga 1980

gtggatttcc accaacattc aaggaaacaa atcaactaaa aacaaaattg ccagaaaatc 2040

tttcctctaa agtcaaactg ttgcagttgt attcagaggc cagtgtagcg cttctaaaac 2100

tgaataaccc caaggatttt caagaattga ataagcaaac taagaagaac atgaccattg 2160

atggaaaaga actgaccata agtcctgcat atttattatg ggatctgagc gccatcagcc 2220

agtctaagca ggatgaagac atctctgcca gtcgttttga agataacgaa gaactgaggt 2280

actcattgcg atctatcgag aggcatgcac catgggttcg gaatattttc attgtcacca 2340

acgggcagat tccatcctgg ctgaaccttg acaatccccg agtgacaata gtaacacacc 2400

aggatgtttt tcgaaatttg agccacttgc ctacctttag ttcacctgct attgaaagtc 2460

acattcatcg catcgaaggg ctgtcccaga agtttattta cctaaatgat gatgtcatgt 2520

ttgggaagga tgtctggcca gatgattttt acagtcactc caaaggccag aaggtttatt 2580

tgacatggcc tgtgccaaac tgtgccgagg gctgcccagg ttcctggatt aaggatggct 2640

attgtgacaa ggcttgtaat aattcagcct gcgattggga tggtggggat tgctctggaa 2700

acagtggagg gagtcgctat attgcaggag gtggaggtac tgggagtatt ggagttggac 2760

agccctggca gtttggtgga ggaataaaca gtgtctctta ctgtaatcag ggatgtgcga 2820

attcctggct cgctgataag ttctgtgacc aagcatgcaa tgtcttgtcc tgtgggtttg 2880

atgctggcga ctgtgggcaa gatcattttc atgaattgta taaagtgatc cttctcccaa 2940

accagactca ctatattatt ccaaaaggtg aatgcctgcc ttatttcagc tttgcagaag 3000

tagccaaaag aggagttgaa ggtgcctata gtgacaatcc aataattcga catgcttcta 3060

ttgccaacaa gtggaaaacc atccacctca taatgcacag tggaatgaat gccaccacaa 3120

tacattttaa tctcacgttt caaaatacaa acgatgaaga gttcaaaatg cagataacag 3180

tggaggtgga cacaaggagg ggaccaaaac tgaattctac agcccagaag ggttacgaaa 3240

atttagttag tcccataaca cttcttccag aggcggaaat cctttttgag gatattccca 3300

aagaaaaacg cttcccgaag tttaagagac atgatgttaa ctcaacaagg agagcccagg 3360

aagaggtgaa aattcccctg gtaaatattt cactccttcc aaaagacgcc cagttgagtc 3420

tcaatacctt ggatttgcaa ctggaacatg gagacatcac tttgaaagga tacaatttgt 3480

ccaagtcagc cttgctgaga tcatttctga tgaactcaca gcatgctaaa ataaaaaatc 3540

aagctataat aacagatgaa acaaatgaca gtttggtggc tccacaggaa aaacaggttc 3600

ataaaagcat cttgccaaac agcttaggag tgtctgaaag attgcagagg ttgacttttc 3660

ctgcagtgag tgtaaaagtg aatggtcatg accaggtcag gaatccaccc ctggacttgg 3720

agaccacagc aagatttaga gtggaaactc acacccaaaa aaccataggc ggaaatgtga 3780

caaaagaaaa gcccccatct ctgattgttc cactggaaag ccagatgaca aaagaaaaga 3840

aaatcacagg gaaagaaaaa gagaacagta gaatggagga aaatgctgaa aatcacatag 3900

gcgttactga agtgttactt ggaagaaagc tgcagcatta cacagatagt tacttgggct 3960

ttttgccatg ggagaaaaaa aagtatttcc aagatcttct cgacgaagaa gagtcattga 4020

agacacaatt ggcatacttc actgatagca aaaatactgg gaggcaacta aaagatacat 4080

ttgcagattc cctcagatat gtaaataaaa ttctaaatag caagtttgga ttcacatcgc 4140

ggaaagtccc tgctcacatg cctcacatga ttgaccggat tgttatgcaa gaactgcaag 4200

atatgttccc tgaagaattt gacaagacgt catttcacaa agtgcgccat tctgaggata 4260

tgcagtttgc cttctcttat ttttattatc tcatgagtgc agtgcagcca ctgaatatat 4320

ctcaagtctt tgatgaagtt gatacagatc aatctggtgt cttgtctgac agagaaatcc 4380

gaacactggc taccagaatt cacgaactgc cgttaagttt gcaggatttg acaggtctgg 4440

aacacatgct aataaattgc tcaaaaatgc ttcctgctga tatcacgcag ctaaataata 4500

ttccaccaac tcaggaatcc tactatgatc ccaacctgcc accggtcact aaaagtctag 4560

taacaaactg taaaccagta actgacaaaa tccacaaagc atataaggac aaaaacaaat 4620

ataggtttga aatcatggga gaagaagaaa tcgcttttaa aatgattcgt accaacgttt 4680

ctcatgtggt tggccagttg gatgacataa gaaaaaaccc taggaagttt gtttgcctga 4740

atgacaacat tgaccacaat cataaagatg ctcagacagt gaaggctgtt ctcagggact 4800

tctatgaatc catgttcccc ataccttccc aatttgaact gccaagagag tatcgaaacc 4860

gtttccttca tatgcatgag ctgcaggaat ggagggctta tcgagacaaa ttgaagtttt 4920

ggacccattg tgtactagca acattgatta tgtttactat attctcattt tttgctgagc 4980

agttaattgc acttaagcgg aagatatttc ccagaaggag gatacacaaa gaagctagtc 5040

ccaatcgaat cagagtatag gtgatagcgg tgtacctcaa ggtctagcca tgtgctaagt 5100

gctctgtaca ctgcttacaa ctgctcaatg gaccagttgt acaagcaggg agctttaagg 5160

tagtgcagat ttacaggatg tcatgcggat catggtgatt gagcttattg ttcaaagagg 5220

catccgtttg gtttgttgga cggaaccgag cagaactcat cgccacaact ttgaactgca 5280

ttactatgta acgatggagg gctctttcga aattatctac actaaaatga aaatttgaaa 5340

tcctcactag acgaagcgca cggcagattc acagctccgt gtgcagccgg tcatgcggta 5400

ttttttttac actcttgttt acaacaggtg ttgtatctgt tacgcgctgc agccacgccg 5460

ccacaagttt cagactgctg ctgcgagaga atgggagagc accccataaa agaaacatag 5520

gaactctgga tcattgactt ctaaagcacc aatttattaa cacatttttt gaaacagcaa 5580

gaagtgtttc tgctattttt ctgttatttc ttttcactgt gcaaagttgg cgttcaagaa 5640

ctataaattt gaattgctga cctatttggt gcaacagcca ataggttaag attggaccaa 5700

aaatcccacc tttaccttga aaaagatttt tttaattctt atttcaaaaa tcattctttt 5760

gtttttcttt ttttttgtgg agattggttt cataaaaaga acataagctt aaacatcagg 5820

ttttactgta tcaagaattt atttaatatg cactagtggt cattacgtag cacgctacga 5880

acaggagctc ggatcc 5896

<210> 2
<211> 2748
<212> DNA
<213> Artificial

<220>
<223> expression construct

<400> 2
ccatggtcta gaccgtcagt cttcctcttc caaaagcctt tacgattaaa atcataaaat 60

aaactgaaaa aatgaaatta tcatgtttta aacaaaacat caattcatat atactagaaa 120

attatttttg aaaccaaaat cattctattt attgcatcga ttattcttct ttcaacaaca 180

caaagttttc aatggaagat catatccata attcattaca ctaaaaaaag agataagtga 240

gtgctaaaat tgatctaata tataaattta atttcaagcc tactttatca tcttataaag 300

attagttgag acattagaaa gttttggatt tttctaccca agttggcttt atttttactt 360

atattcatat ccactcgttt aatgtgcaaa gaaatatttt tagcaacata aatgcatacg 420

aaaacaaaac aaacacataa acaaacaaaa cttattaata catgatttta tcaaagaatg 480

acttttgtac ctttcaatgg gaacaggacc atcgagaaag ccaagtggaa acagggccgt 540

cgaggcagcg tccacgtcag cgtatgatta ttccgcggca tgcgaacgtg gcatacatga 600

cgtggcgccg gcgaagcttc cgaagggaat cgaactgcgg tgggaacaag acctgacata 660

agagtccacg tcagataatt ttaatgctgc cggtggtgat gtggcaggga tgacatggca 720

ccggctgagc cagcgatcgg gttcgtctcg agccctactt tctccgcttt cgtgcaaaat 780

tcgtttgcca gggagactcg attcacagaa acggtccaca tgatccggcc agggaatcag 840

ttaccccctt cacccccttc ggcgttgctt gtggatcatt tcgtgattaa cgcgagcact 900

ggctaggaat actgttttgg tcatttgatt tgcggagagc aatcggtgtc atggaaaatc 960

ccttcgactg ctttgatcca atcaaagttc tgcttgagcc aatgtgagag gtggaggatt 1020

gggcttcttc taagtgaggg ctttcgatta ttgatatctc aaggcgaatg ttgaaggcgc 1080

ttagggagta aatatggctt tctataagat ttcatctgtt tttttcatct tctgtttctt 1140

cttgattgcc cttccgttcc actcctatgc cgcgaagatg aaggtggtgg aggagcccaa 1200

cgcgtttggg gtgaacaacc cgttcttgcc tcaggccagt cgcctccagg ccaagaggga 1260

tccttcaccc gtgtctggac ccgtgcatct cttccgactc tcgggcaagt gcttcagcct 1320

ggtggagtcc acgtacaagt atgagttctg cccgttccac aacgtgaccc agcacgagca 1380

gaccttccgc tggaacgcct acagtgggat cctcggcatc tggcacgagt gggagatcgc 1440

caacaacacc ttcacgggca tgtggatgag ggacggtgac gcctgccgtt cccggagccg 1500

gcagagcaag gtggagctgg cgtgtggaaa aagcaaccgg ctggcccatg tgtccgagcc 1560

gagcacctgc gtctacgcgc tgacgttcga gacccccctc gtctgccacc cccacgcctt 1620

gctagtgtac ccaaccctgc cagaggccct gcagcggcag tgggaccagg tagagcagga 1680

cctggccgat gagctgatca ccccccaggg ccatgagaag ttgctgagga cactttttga 1740

ggatgctggc tacttaaaga ccccagaaga aaatgaaccc acccagctgg agggaggtcc 1800

tgacagcttg gggtttgaga ccctggaaaa ctgcaggaag gctcataaag aactctcaaa 1860

ggagatcaaa aggctgaaag gtttgctcac ccagcacggc atcccctaca cgaggcccac 1920

agaaacttcc aacttggagc acttgggcca cgagacgccc agagccaagt ctccagagca 1980

gctgcggggt gacccaggac tgcgtgggag tttgtgatcc cgttttcata tcagtgtatt 2040

atcatcagtg actgcatatt gacacccaat tctgatgatt ttttattttt tattttttat 2100

tttttttggt atggttacat gcttttcaga ggtttctatg ccgctgagta ttttcctgaa 2160

tcgcgaggtg tggcaggtta tctgcgccgt ccacccaata ttttatgatg agtcgatgat 2220

tcgtgagact aatctagctt aacctttttc ttactggcaa gtcaaaattg agtttaaaat 2280

atttcagtat cctgttagta atttcagaca catgtattct atgtctcata ctctttacgt 2340

gaaagttcaa ctgacttata ttttgtcgtt tttctgtaga tcactgtttt agcgcataca 2400

aagacaattg tctaaatatt tttaaagaag gtgatatttt attataagat agaagtcaat 2460

atgttttttt gttatgcaca tgtacttgaa taaaataaat ttttttgtta gatttaaata 2520

ctttttgaat tatagctttg ttgaaattaa ggaatttata ttcataagaa gctactcgaa 2580

caaatttaca aagagaacat ttgataagta aaagtaatta aaagtttttt ttaatttaaa 2640

aagattaatt tttattaata agaaaaattt ggaaagttag aaaaatattt aactttaaaa 2700

attaagaaaa caaggcaggt ctccaacaaa gccctcgaat tcccatgg 2748

<210> 3
<211> 3293
<212> DNA

<213>  Artificial

<220>
<223>  expression construct

<400>  3
ccatggctcg agcgccaaga actcactgta tcagggaagg ggagcgaggc agagcagaga      60

gacccactga gagagacaca cacacacaca cacagagcag agagatagca gcagaggggg     120

agcgcagaga gcagagtgta gtggacagag agccctgagt cagacagaca gacaggccca     180

cacgtacaca cagacagagc aagaatggca cacagaaata tatctatcta tatgtaaata     240

tatccgcgac ggacggcccc ctccctcctc ctcccccgcg cgcactcact tgggttattt     300

tacaattatt attattatta ctattcttcc aaaggaagag aaggaaagcg aagcgagggg     360

aggggaatat aatatcatga agaagaagac atgggagtcg gttgtcagtt ggatgaataa     420

taatcaatcc ccagtttggc ttcttctgct tcttcttctt ttcacatggg tgttgtgccc     480

ttttttgact ctctctctac cgttctggtc ctggtcctgg gccttgagga ggtgcacact     540

ctacttcctt cccttccttc ccttctcttc cttcccttcc cttcctcctc cttattgatt     600

gtttgctttg tgaagaagag tatgttagtt gcacgatagg cgatttagcg tcactgcgag     660

cgttcgggct gtgtagaagg gaccgatgat tgccgtagca ggagattttc aagaagatcg     720

tcaactctgc gcatatgtgt gcctcggggg tttgagtcaa tgtgactgat aacggggtga     780

ataaatattc ggaagtagca tctagggctt cacctcaatt aggtcattgc cagcgctaca     840

ggagcaataa gaaaggccag agtaacggag acggcctact tattctagat atcattctac     900

acggcctact aattttctgg tttctagata tctcggtttc aacactgttg gactcataca     960

gcaaaatata caactgtgtg gacattgttt gtctgacaaa cagagcaatc tgcattgaat    1020

gtttgtcggt ggcagtgata cagatggttg tatttctgag gtggtgctta ttgatggtgg    1080

aggtgatggt acaaattatt gacattgcat gaatattatc gggctagttc gtgtggagat    1140

cattcggtca aggttgagca atcatatggc caagtgtttg taggaaaaag tttcggagat    1200

ggctttctat aagatttcat ctgttttttt catcttctgt ttcttcttga ttgcccttcc    1260

gttccactcc tatgccggcc tcgactcggg ggcctcccgc gacgacgact tgctactgcc    1320

ctatccacgc gcgcgcgcgc gcctcccccg ggactgcaca cgggtgcgcg ccggcaaccg    1380

cgagcacgag agttggcctc cgcctcccgc gactcccggc gccggcggtc tggccgtgcg    1440

caccttcgtg tcgcacttca gggaccgcgc ggtggccggc cacctgacgc gggccgttga    1500

gcccctgcgc accttctcgg tgctggagcc cggtggaccc ggcggctgcg cggcgagacg    1560

acgcgccacc gtggaggaga cggcgcgggc ggccgactgc cgtgtcgccc agaacggcgg    1620

cttcttccgc atgaactcgg gcgagtgcct ggggaacgtg gtgagcgacg agcggcgggt    1680

gagcagctcc ggggggctgc agaacgcgca gttcgggatc cgccgcgacg ggaccctggt    1740

caccgggtac ctgtctgagg aggaggtgct ggacactgag aacccatttg tgcagctgct    1800

gagtggggtc gtgtggctga ttcgtaatgg aagcatctac atcaacgaga gccaagccac 1860

agagtgtgac gagacacagg agacaggttc ctttagcaaa tttgtgaatg tgatatcagc 1920

caggacggcc attggccacg accggaaagg gcagctggtg ctctttcatg cagacggcca 1980

aacggagcag cgtggcatca acctgtggga aatggcggag ttcctgctga aacaggacgt 2040

ggtcaacgcc atcaacctgg atgggggtgg ctctgccacc tttgtgctca acgggacctt 2100

ggccagttac ccgtcagatc actgccagga caacatgtgg cgctgtcccc gccaagtgtc 2160

caccgtggtg tgtgtgcacg aaccccgctg ccagccgcct gactgccacg gccacgggac 2220

ctgcgtggac gggcactgcc aatgcaccgg gcacttctgg cggggtcccg gctgtgatga 2280

gctggactgt ggcccctcta actgcagcca gcacggactg tgcacggaga ccggctgccg 2340

ctgtgatgcc ggatggaccg ggtccaactg cagtgaagag tgtccccttg gctggcatgg 2400

gccgggctgc cagaggcctt gtaagtgtga gcaccattgt ccctgtgacc ccaagactgg 2460

caactgcagc gtctccagag taaagcagtg tctccagcca cctgaagcca ccctgagggc 2520

gcgaagattc tcttccgcac agcagctgta tcaactcttc ttcctactct tcgccatcat 2580

ctgctgggca tacacgggag cagagctgta gaagctcgtt tccaaagatt attagtcatt 2640

ctttaagttg cttcgattgc ttagtagcag aagcgtccag attttggatc gtggggggag 2700

agtttgttca agttggatta ccagagtcgg ctaagtctac cttttggaag agtataattg 2760

ttaccaggta aatatttctt acaaggatag cagatgacat cttgtatcgg ctccgtccac 2820

ttacacaaat atgtttataa cagttagagg gtcttggagt gtagctgaga gttggcttga 2880

cctacagaag ctcgtgtgtt aatcagaagc aagctctgcc attgtgccta gtgaagtgca 2940

gtggtgtttc ggaatattat gtatcctgtc gtgccacacg ctatttgctg aagtgttgca 3000

gtatgtagtg tcattgatca catgagaccc tgaacaagac tatctaattg cagggcataa 3060

ctccatagac acatttgcat cactggtacg ctggtcaccg agatttgcta aatcttattc 3120

aagaacaatg taatgctaga cgcacttcat attgacatgc tattcctgta tttgatttca 3180

gtgattgaga cattgtcact ctgaaatttc gggagcatta gatatttctt acacctttac 3240

accttctaag tactctgcac agtctcctca gctagcacca aaagcttcca tgg 3293

<210> 4
<211> 2958
<212> DNA
<213> Artificial

<220>
<223> expression construct

<400> 4
ccatggctcg agccactcct atgccaatag tgactcactt gtaagttatt aaaaggaaag 60

acatggccat ttcttattac aacatccatt caagcaaaag aattcatggc atgatttgag 120

gacgtggttg ttttcatggg acaaaggaga tccacacatg gaaagaaaac cacacttcag 180

tattacagaa atagcaagtc tcacgaatta gtgtgaactt tcaacgctac gatggctgca 240

catgtcatcc gccactcatc cgaccatcca tcatccatcc acacacagtt ccggccatca 300

aagggatcgc cacgtcacga tccggacagc acagagaggc tcgtcacgtg gcataagtcg 360

tccttctgga caacgaagta caacgtacgt cagatgtggc gacgctgacc ctcaagaacg 420

agacgatata ctgattctcg ccgacggaga ccagaacggg attccttctc gtctccctgg 480

cggacgatgt cgcgacccag ccgtgcttaa cagagtggct ggctggctct gttgctgaag 540

aagctgatgc tgctgctggc ggattgtgga tgtgggtgca tgctcggcga ttcatcacag 600

atgtgagcgc aaccccagtc actattcacc cacagccaaa gattcctgaa attttctgtg 660

aaggagcggt gatgaggtgg agagattgaa gcggggcacc atgtcgggga cgtgttgtag 720

gccatgttgc aacttcggga agcgttgtga gattttccca atggttagtc acttatctgc 780

ttaaagcgtt tttcatgccg tgccagtgct ccgccgatga tggtgaagca tcgtcgccga 840

atgggcggtg tgtcgccggc gacatggctg ctgctctcgc tccccgctct cctctgcttt 900

gcgggcttgc cgttcatgct cagctcgccc cagatgtctg aacagtatgt tacaccatac 960

cttccgaaat ctttgcaacc tattgcaaaa attagtgcag aggaacaaag gcgtatacaa 1020

agtgaacagg aagaagccga attgaagcaa tctttagagg gagaggccat tagaaatgcc 1080

accgtgaatg ccattaagga aaagatcaaa tcttatggtg gtaatgaaac gacgctaggg 1140

ttcatggtgc catcgtatat caatcataga ggatcaccac caaaggcgtg ctttgtctca 1200

ctaattactg aaagggattc catgacccaa atcttacaat ctattgatga agtgcaggtg 1260

aagtttaaca aaaattttgc ttatccttgg gtttttatta gtcaggggga gcttgacgga 1320

atgaagcaag aaatgattcg tcaagccata actgattcta tgaatggtga tcctgagtta 1380

attaatatta aatttgctga aattcctgca gacgaatggg tataccctga atggattgat 1440

gaaaataaag cagcagaatc gctaatttca ttagcaaatg taccggatgg tgattctaga 1500

gctgtaagat atcaagctag atattttgca gggttttttt ggaggcatcc agttctggat 1560

gagtttgatt ggtactggag agtagaccct ggtattaaat tgtactgtga tattgatcat 1620

gaccttttta ggtggatgca agatgaaggc aaagtgtttg gtttcacatt gagtatgagt 1680

gaagctaagg aagccaatga gaagatttgg gatgttacga aaaaatttgc aaaggatttt 1740

ccaaagttta tttccgagaa taatttcaag tcatttatta caaaaaagga ctctgaagat 1800

tttaacaact gtgaattcac atcaaatttt gaaattggta atttgaactt ttatagatcg 1860

ccagcttaca ggaaattttt taattacatc gacgaagagg gcggtatttt ctactggaaa 1920

tggtctgatt ccatcatcca cacaattggg ttatcgatgc tgttgccaaa ggataaaata 1980

cattttttcg aaaatatagg attccattat gacaagtaca ataactgtcc cctaaacgat 2040

gatatatgga accaatataa ttgtaactgt gaccagggta acgatttcac gtttagaagt 2100

ggttcatgtg gcggacatta cttcgacatt atgaagaaag ataagccaga aggttgggat 2160

aggttaccat aaggtgaaac ctgtgaaaat tactatttca accaatgaaa gaaggtaaca 2220

aagttccttg gaggagcgaa tcaaggatcg ccgagcagga tcaaatgacg ggaatgttgt 2280

gatggtcatg caacaatcca attcactagt ggcaacgcag gtgaattgaa ttccagaggg 2340

aaagagaggc agcatgcaga tgtttcggat agtatcagga cgaaaaagat gcacaattat 2400

cgggcaaatg atgcgctatt gctgcattgt taagcgctct ggttggtgtg tatgttgcag 2460

ctgcaagaaa acaagaatgt ggcgcctgcc taattctctg gccacaatcg gccagtattg 2520

gaggcattgc gttatttgta ccaatgtcgg ttgcgtcgca gattctcctt tttgctgtcg 2580

ccttgactta tcatttaatt tattcactct gagtcctctc aatgagttcc atatttaaca 2640

agggttctgt ttacttgtgg agcaaccagt tgtaaggtag agtagttttg tgtctgggga 2700

gagagttcat atactcaaca cgatatagat agttgtgaag tgtagcagtt gagaataggt 2760

tatgagagat cacacccaga caaacgacgt gttgggaata aagttaactg cttaaagaat 2820

tacttcgaag ctgaattaag gcggtttgag gagaaatgcc caacgtagta actggcatgg 2880

gctgcagacg tcgatgtcgt caatgcaacg atcaactttt cacattacgt agcacgctac 2940

gaacagaagc ttccatgg 2958

<210> 5
<211> 22
<212> DNA
<213> Artificial

<220>
<223> primer

<400> 5
gccaatgcac aagtttgaca tc 22

<210> 6
<211> 24
<212> DNA
<213> Artificial

<220>
<223> primer

<400> 6
ccactcctat gccaatagtg actc 24

<210> 7
<211> 22
<212> DNA
<213> Artificial

<220>
<223> primer

<400> 7

gtagccaaaa aacttagcca ac                                                22

<210>  8
<211>  24
<212>  DNA
<213>  Artificial

<220>
<223>  primer

<400>  8
cccaggtgta aacaacgtca atcg                                              24

<210>  9
<211>  18
<212>  DNA
<213>  Artificial

<220>
<223>  primer

<400>  9
catgcgaacg tggcatac                                                     18

<210>  10
<211>  19
<212>  DNA
<213>  Artificial

<220>
<223>  primer

<400>  10
ccgtcagtct tcctcttcc                                                    19

<210>  11
<211>  21
<212>  DNA
<213>  Artificial

<220>
<223>  primer

<400>  11
agtaagatgg agtcacatta g                                                 21

<210>  12
<211>  19
<212>  DNA
<213>  Artificial

<220>
<223>  primer

<400>  12
ctcctccacc accttcatc                                                    19

<210>  13
<211>  19

<212>  DNA
<213>  Artificial

<220>
<223>  primer

<400>  13
gtcggttgtc agttggatg                                                    19


<210>  14
<211>  20
<212>  DNA
<213>  Artificial

<220>
<223>  primer

<400>  14
cgccaagaac tcactgtatc                                                   20


<210>  15
<211>  22
<212>  DNA
<213>  Artificial

<220>
<223>  primer

<400>  15
gggaagaaga gaagcagaga ag                                                22


<210>  16
<211>  19
<212>  DNA
<213>  Artificial

<220>
<223>  primer

<400>  16
atagggcagt agcaagtcg                                                    19


<210>  17
<211>  18
<212>  DNA
<213>  Artificial

<220>
<223>  primer

<400>  17
tggcataagt cgtccttc                                                     18


<210>  18
<211>  20
<212>  DNA
<213>  Artificial

<220>
<223>  primer

<400>  18
gatgcttcag ccatctaacc                                                   20

<210>  19
<211>  20
<212>  DNA
<213>  Artificial

<220>
<223>  primer

<400>  19
cctagcgtcg tttcattacc                                                   20

## Description

The present invention relates to artificially modified glycosylation pathways for the post-transcriptional modification of glycoproteins in plants.

### Background

Glycosylation is a form of co-translational and post-translational modification of proteins. Glycan modifications serve important functions including correct folding, stability and receptor-ligand interactions. Bacteria do not glycosylate proteins, so glycoproteins cannot be produced in these organisms. The glycosylation mechanisms in the higher organisms are evolutionarily conserved but differ in detail. Yeast, insect, mammalian and plant cells all attach high-mannose glycans to the same Asn residue, but differ in the trimming and further modification of the glycans in the Golgi. Thus no heterologous system will reproduce the mammalian glycans exactly. Because plants are well suited for the production of large amounts of glycoproteins at low cost, they have gained acceptance for the expression of recombinant therapeutic proteins. It is therefore an ongoing goal to minimize or eliminate these differences.

For example, WO00/49153, WO01/64901 and WO 2004/057002 describe the identification and inhibition of plant specific alpha-1,3-fucosyltransferase and beta-1,2-xylosyltransferase, providing the first stepping stones towards the expression of proteins with mammalian-type glycosylation in plants. Other differences still exist.

Some mammalian proteins require mannose-6-phosphorylation (M6P) on their N-glycans for correct targeting to their site of action. The recognition and subsequent uptake into the target cell is mediated by so called M6P-receptor present on cell surfaces. Plant-made, recombinant versions of these proteins lack the M6P-signal as plants are devoid of a functional mannose-6-phosphorylation pathway. Despite offering a good set of advantages, plants are not yet considered as a production host for such therapeutic proteins carrying the M6P-marker as the products would remain ineffective. Examples for M6P-mediated uptake of recombinant therapies include alpha-galactosidase (*Fabry* disease) and acid alpha-glucosidase (Pompe disease).

The publication US 2003/0133924 describes the use of isolated GlcNAc phosphotransferase for an *in vitro* modification of acid beta-glucocerebrosidase, which in turn can be used in the treatment of Gaucher disease. Acid beta-glucocerebrosidase can be expressed in mammalian CHO cells with mannosidase-1 inhibitors to obtain high-mannose N-glycan structures, which is then phosphorylated *in vitro.*

Akeboshi et al. (Glycobiology 19(9), 2009: 1002-1009) describe the production of human β-hexosaminidase A with highly phosphorylated N-glycans by the overexpression of the *Ogataea minuta* MNN4 gene in yeast.

Tiels et al. (Nature biotechnology 30(12), 2012) describe a method for increasing mannose-6-phosphate modification of lysosomal enzymes produced in yeast. A glycosidase from C. cellulans was identified that 'uncaps' N-glycans modified by yeast-type mannose-P6-mannose to generate mammalian-type N-glycans with a mannose-6-phosphate substitution.

### Summary of the invention

It is a goal of the present invention to allow production of mannose-6-phosphate modified glycoproteins in plants.

The invention allows to genetically engineer the M6P-pathway into plants and thereby enables the recombinant production of functional M6P-proteins in these species.

Thus in a first aspect of the present invention, a recombinant plant cell is provided comprising mannose-6-phosphate modification activity. These plants can be used in methods to produce glycoproteins comprising a mannose-6-phosphate glycosylation. These uses and methods constitute another aspect of the invention. Also provided are methods to produce such recombinant plant cells by introducing genes of the mannose-6-phosphate pathway into said plants. Even further provided are genetic constructs that comprise genes of the mannose-6-phosphate pathway suitable for plant transformation. All these aspects are interrelated, equally form part of the entire invention presented herein and preferred embodiments of the invention in any combination may relate to any one of these aspects, e.g. plants transformed by a given construct or method can be provided or used in the production of any glycoprotein to be mannose-6-phosphorylated. The present invention is further defined in the claims.

In particular, the present invention provides a method of expressing a glycoprotein with a mammalian-type phosphoglycosylation in a plant cell comprising expressing said glycoprotein in a plant cell, wherein said plant cell recombinantly expresses
i) N-acetylglucosamine-1-phosphotransferase (GNPT) and N-acetylglucosamine-1-phosphodiester-alpha-N-acetylglucosaminidase (NAGPA), or
ii) mannosyl-phosphate-transferase Mnn6p.

Also provided is a plant cell comprising recombinant genes for one or more enzymes selected from
i) N-acetylglucosamine-1-phosphotransferase (GNPT) and N-acetylglucosamine-1-phosphodiester-alpha-N-acetylglucosaminidase (NAGPA), or
ii) mannosyl-phosphate-transferase Mnn6p.

Even further provided is a genetic expression construct comprising a coding sequence of an enzyme selected from
a) N-acetylglucosamine-1-phosphotransferase (GNPT) or any one of its subunits,
b) N-acetylglucosamine-1-phosphodiester-alpha-N-acetylglucosaminidase (NAGPA), and
c) mannosyl-phosphate-transferase Mnn6p,
wherein said construct comprises a plant golgi signal sequence operatively linked to said enzyme coding sequence and/or a transmembrane sequence as part of said coding sequence operatively linked to said enzyme coding sequence, and a plant promoter sequence. Such construct, especially a) and b), may be provided in a kit.

Also provided is a method of producing the plant cell according to the invention comprising transforming said plant cell by introducing one or more constructs, optionally in combination such as a) and b), into a plant cell.

### Detailed description of the invention

The present invention provides according to one embodiment recombinant plants or plant cells, which co-express recombinant enzymes GNPT and NAGPA, e.g. each of a mammal, as well as constructs for the respective transformation of plants or plant cells and the use of these plants or plant cells in the expression of a glycoprotein. It was found that the mammalian enzymes GNPT and NAGPA can be used in a plant expression system to complete the mannose-6-phosphorylation pathway. Expression comprises three genes, GNPTAB, GNPTG and NAGPA, which are coding for alpha, beta (GNPTAB as one precursor), gamma subunit (GNPTG) of GNPT and NAGPA, respectively. These enzymes add GlcNAc-phosphate residues to N-glycan mannoses (GNPT) and subsequently remove the GlcNAc moiety (NAGPA), leaving the phosphate on the N-glycan mannose uncovered. GNPT subunits may be expressed recombinantly using coding sequences of GNPTAB and GNPTG genes, the proteins of which form the complete GNPT complex.

A GNPTAB sequence coding for the alpha- and beta- subunits of GNPT is e.g. provided by the sequence of nt (nucleotides) 1422-5057 of SEQ ID NO. 1. A GNPTG sequence coding for the gamma-subunit of GNPT is e.g. provided by the sequence of nt 1172-2014 of SEQ ID NO. 2. The present invention provides the use of these coding sequences according to any aspect, or homologous sequences thereof encoding an active GNPT with at least 50%, at least 60%, at least 70% at least 80%, at least 85%, at least 90%, at least 95% or at least 98% sequence identity to any one of these coding sequences. Such GNPT sequences are well known in the art, e.g. from various mammalian sources, such as a human, and are accessible in sequence databases such as of the NCBI or of the EBI. Homologous sequences can be found using name or sequence searches, e.g. using standard programs at standard parameters such as FastDB or BLAST.

A NAGPA coding sequence is e.g. provided by the sequence of nt 1277-2521 of SEQ ID NO. 3. The present invention provides the use of this coding sequences according to any aspect, or homologous sequences thereof encoding an active NAGPA with at least 50%, at least 60%, at least 70% at least 80%, at least 85%, at least 90%, at least 95% or at least 98% sequence identity to said coding sequence. Such NAGPA sequences are well known in the art, e.g. from various mammalian sources, such as a human, and are accessible in sequence databases such as of the NCBI or of the EBI. Homologous sequences can be found using name or sequence searches, e.g. using standard programs at standard parameters such as FastDB or BLAST.

In a preferred embodiment, said plant cell further has a reduced activity, preferably a complete loss of function, of GlcNAc-transferase I (GnTI), in particular by knock-out, especially preferred by interrupting the GnTI gene of said plant, even more preferred by a gene of any one of the recombinantly expressed proteins or a separately expressed subunit thereof, such as GNPT-alpha, -beta and/or -gamma. The plant GlcNAc-transferase I (GnTI) can be removed from the plant expression system or reduced in activity at least in the golgi. This enzyme normally transfers GlcNAc residues to a certain mannose on N-glycan chains to initiate complex glycan formation. Lack of GnTI activity blocks complex glycan formation leading to increased amounts of high-mannose N-glycan chains on proteins, which act as substrates for mannose-6-phosphorylation by GNPT. Glycosylation is a competitive process, wherein various enzymes compete for the same or similar substrates. Thus by removing one competitor the activity of the other competing enzyme can be increased.

According to a second embodiment the invention provides recombinant plants or plant cells which express the enzyme MNN6P, e.g. from yeast, as well as constructs for the respective transformation of plants or plant cells and the use of these plants or plant cells in the expression of a glycoprotein. As an alternative solution, the yeast mannosyl-phosphate transferase Mnn6p is expressed in plants to enable mannose-phosphorylation of proteins. This enzyme adds mannosyl-phosphate moieties to 1,2-linked mannose on glycan structures.

A mannosyl-phosphate transferase Mnn6p coding sequence is e.g. provided by the sequence of nt 931-2269 of SEQ ID NO. 4. The present invention provides the use of this coding sequences according to any aspect, or homologous sequences thereof encoding an active mannosyl-phosphate transferase Mnn6p with at least 50%, at least 60%, at least 70% at least 80%, at least 85%, at least 90%, at least 95% or at least 98% sequence identity to said coding sequence. Such mannosyl-phosphate transferase Mnn6p sequences are well known in the art, e.g. from various yeast sources, such as a *S. cerevisiae* or Ogataea, and are accessible in sequence databases such as of the NCBI or of the EBI. Homologous sequences can be found using name or sequence searches, e.g. using standard programs at standard parameters such as FastDB or BLAST.

Sequence identities for any sequence as given herein may also relate to sequence identities on the amino acid level according to the encoded proteins. E.g. the homologous sequence may encode a protein with at least 50%, at least 60%, at least 70% at least 80%, at least 85%, at least 90%, at least 95% or at least 98% sequence identity to any one of the above mentioned enzymes encoded by the nucleotide portions given above for SEQ ID NO. 1-4.

The coding sequences used for the recombinant transformation of the plant cell or in a construct may be genomic sequences (including introns that can be removed in the plant cell) or cDNA.

Suitable transformation systems have been developed for the biotechnological exploitation of bryophyta for the production of heterologous proteins. For example, successful transformations have been carried out by direct DNA transfer into protonema tissue using particle guns. PEG-mediated DNA transfer into moss protoplasts has also been successfully achieved. The PEG-mediated transformation method has been described many times for *Physcomitrella patens* and leads both to transient and to stable transformants (K. Reutter and R. Reski, Pl. Tissue culture and Biotech., 2, 142-147 (1996)). Moreover, marker-free transformation can be achieved by PEG-mediated transformation method with bryophytes as well (Stemmer C, Koch A and Gorr G (2004), Moss 2004, The 7th Annual Moss International Conference, Freiburg, Germany) and can be used for subsequent introduction of multiple nucleotide sequences.

Detailed information on the culturing of bryophytes which are suitable for use in the invention, such as *Leptobryum pyriforme* and *Sphagnum magellanicum* in bioreactors, is known in the prior art (E. Wilbert, "Biotechnological studies concerning the mass culture of mosses with particular consideration of the arachidonic acid metabolism", Ph.D. thesis, University of Mainz (1991); H. Rudolph and S. Rasmussen, Crypt. Bot., 3, 67-73 (1992)). Especially preferred for the purposes of the present invention is the use of *Physcomitrella patens,* since molecular biology techniques are practiced on this organism (R. Reski Bot. Acta, 111, pp. 1-15 (1998)). For cultivation of bryophytes media with (Baur et al . (2005) Plant Biotechnol J 3, 331-340) or without supplements like trace elements can be used (Weise et al (2006) Appl . Microbiol. Biotechnol., 70, 337-345).

According to a preferred embodiment, said plant cell further has a reduced activity, preferably a complete loss of function, of mannosidase I (MnsI), in particular by knock-out mutation of an endogenous MnsI gene, especially preferred by interrupting the MnsI gene of said plant cell, even more preferred by a gene of any one of the recombinantly expressed proteins, or by administering a mannosidase inhibitor to said plant cell. The plant mannosidase I (MnsI), which normally removes 1,2-linked mannose from N-glycan chains during trimming can be mutated to reduce activity or to a loss-of-function version of the enzyme in order to increase mannose-6-phosphate modification - at least in the golgi. Mannosidase I activity can also be reduced by use of a mannosidase I inhibitor such as disclosed in US 2003/0133924.

It is further possible to treat proteins produced in this plant expression system with glycosidase to remove the added mannose moieties, leaving the phosphate on the N-glycan mannoses uncovered. The glycosidase can be a recombinant glycosidase.

It is also possible to upregulate Mnn6p by expression of Mnn4p, which can be over-expressed (Akeboshi et al., supra).

Of course aspects of this first and second embodiment can be combined, e.g. the plant may express GNPT, NAGPA and Mnn6p, and/or a GNPT and NAGPA transformed plant or cell may have reduced MnsI activity, and/or the produced glycoproteins may be treated with a glycosidase; also a Mnn6p transformed plant or cell may be further modified to reduce the activity of GnTI.

Thus, the invention relates to the use of heterologous mammalian or yeast enzymes in plants. So far these enzymes have only been used in their native host environment. Introducing these enzymes in plants is a new step, providing the plant cells with a formerly not achievable N-glycan modification, the mannose-6 phosphorylation, a modification completely foreign to plants.

Other plant enzymes can be reduced in activity or concentration, at least in the site of their natural activity such as the golgi. Further enzymes that are preferably removed are alpha-1,3-fucosyltransferase and/or beta-1,2-xylosyltransferase as described in WO 2004/057002. Thus according to a preferred embodiment, said plant cell further has a reduced activity, preferably a complete loss of function, of alpha-1,3-fucosyltransferase and/or beta-1,2-xylosyltransferase, in particular by knock-out, especially preferred by interrupting the alpha-1,3-fucosyltransferase and/or beta-1,2-xylosyltransferase encoding gene of said plant, preferably by a gene of any one of the recombinantly expressed proteins. This measure prevents formation of plant-type glycosylations that may be immunogenic in a mammal such as a human.

The method of the invention may comprise introducing a functional galactosyltransferase (GalT), for example a mammalian beta-1,4 galactosyltransferase, preferably a human beta 1,4 galactosyltransferase nucleotide sequence into the transformed plant cell of the invention. The resulting plant cell may have increased GalT activity.

Increased or reduced activity or concentration as used herein refers to an increase or reduction in activity or protein concentration in a transgenic plant due to the mutations incurred by the present invention as compared to a wild-type plant or plant cell of the same plant species. Comparisons are made at the same or similar stage of development and/or culture conditions in order to remove influences not related to the genetic transformation. Reductions in activity are preferably by at least 50%, by at least 80% or at least 90%, preferably complete ablations in activity. Such reductions can be achieved by knock-out or knock-down mutations, e.g. by RNAi and antisense technology.

To allow proper localization of the transgenes, the genes for any one of these enzymes can be modified to allow for modified localization in the plant cell. Thus preferably hybrid enzymes regarding the origin of their domains are used in the constructs for the transformation of the plants or plant cells. Localization-relevant domains of the yeast/mammalian enzymes are replaced by plant sequences to achieve correct localization such as in the ER and/or golgi *in planta.*

Moreover, the first and second embodiments may be combined by introduction of the new enzymes with simultaneous, targeted knock-out of plant glycan-modifying enzymes that are not desired, e.g. alpha-1,3-fucosyltransferase, beta-1,2-xylosyltransferase, GnTI, MnsI or any combination thereof.

The plant may be any plant but is preferably selected from bryophyta, preferably moss, liverwort or hornwort, especially preferred of the class *bryopsida* or of the genera *Physcomitrella, Funaria, Sphagnum, Ceratodon, Marchantia* and *Sphaerocarpos,* or aquatic plant, preferably of the genus *Lemna, Spirodela, Landoltia, Wolffia* or *Wolffiella. Physcomitrella patens* is a particularly preferred system as it has a high rate of homologous recombination. This ensures direction of the glycan-biosynthesis pathway towards mannose-6 phosphorylated N-glycans. Of course other suitable plants exist such as *Lemna,* especially *Lemna minor* (Cox et al., Nature Biotechnology 24(12), 2006: 1591-7).

Subject matter of the invention are plants and plant cells. "Plant cell" as used herein may refer to an isolated cell, a singularized cell, but also a cell in or of a plant tissue, preferably a tissue selected from callus, protonema, phloem, xylem, mesophyll, trunk, leaves, thallus, chloronema, caulonema, rhizoid or gametophore, or a cell in a plant organism. The cell may be a protoplast cell. In preferred embodiments, isolated plant cells or even plant tissues are transformed according to the invention and then grown into plants or plant tissues, or remain plant cell cultures, such as a suspension culture, e.g. a bioreactor (Hohe & Reski, Plant Cell, Tissue and Organ Culture 81, 2005: 307-311).

The glycoprotein may be any glycoprotein intended for mannose phosphorylation. It may be a protein that is naturally (in its native state when expressed in its native host) mannose-phosphorylated or non-naturally mannose-phosphorylated. Example glycoproteins are alpha-galactosidase, acid alpha-glucosidase, beta-hexosaminidase A, arylsulfatase A, arylsulfatase B, lysosomal alpha-mannosidase, L-asparaginase, lysosomal acid lipase, acid ceramidase, beta-galactosidase, alpha-L-fucosidase, alpha-L-iduronidase, iduronate sulfatase, N-acetylglucosamine 6-sulfatase, beta-glucuronidase, Sphingomyelin phosphodiesterase, alpha-N-acetylgalactosaminidase, palmitoyl-protein thioesterase, tripeptidyl-peptidase, ceroid-lipofuscinosis neuronal protein 5 and cathepsin D. A recombinant gene of the glycoprotein may be introduced as is explained herein for the inventive enzymes that modify the glycan phosphorylation pathway.

In certain embodiments it is possible that the glycan terminates with a sugar-phosphate-sugar structure, wherein one or both sugars may be mannose. It may be necessary to remove the terminal sugar to uncover the phosphate. This can be done with a glycosidase as e.g. described in Tiels et al. (supra). Thus the inventive method may further comprise the step of removing a terminal (non-reducing end) sugar residue, preferably mannose, lacking a 6-phosphate, preferably by treating the expressed glycoprotein with a glycosidase. The glycosidase treatment may be *in vivo* (e.g. by introducing a glycosidase into the plant cell, preferably recombinantly) or *in vitro.*

Also provided is a genetic construct, i.e. a nucleic acid molecule, with the following elements a coding sequence of an enzyme selected from N-acetylglucosamine-1-phosphotransferase (GNPT) or any one of its subunits, N-acetylglucosamine-1-phosphodiester-alpha-N-acetylglucosaminidase (NAGPA), or mannosyl-phosphate-transferase Mnn6p. When introduced into the cell, said cell comprises the elements, transiently or stably, e.g. integrated into the cells genome. The sequence in the construct or cell may comprise a plant golgi signal sequence operatively linked to said enzyme coding sequence and/or a transmembrane sequence as part of said coding sequence operatively linked to said enzyme coding sequence. It may also contain a plant promoter sequence operatively linked to said enzyme coding sequence.

The template nucleic acid sequences for the non-plant transgenes for the GNPT, NAGPA or mannosyl-phosphate-transferase Mnn6p may comprise non-plant signal sequences (which may in their native hosts be removed or not during processing). Such non-plant signal sequences may not be functional in the plant. They may remain or be removed from the coding sequence used according to the present invention. Thus "coding sequence" for GNPT, NAGPA or mannosyl-phosphate-transferase Mnn6p only requires the presence of sequences of the enzyme but not of the native signal sequence. Other signal sequences may be added to these parts of the enzyme coding sequence, which are also expressed and within the open reading frame. Such signal sequences may be host plant specific. Example signal sequences are provided in nt 1290-1421 of SEQ ID NO. 1, nt 1094-1171 of SEQ ID NO. 2, 1199-1276 of SEQ ID NO. 3, nt 817-930 of SEQ ID NO: 4. Any one of these signal sequences may be used for any one of the recombinant enzymes to be expressed in plant cells.

For ER and/or golgi localization, the coding sequences may also comprise a transmembrane domain sequence. Such a domain may be included in the heterologous sequences or it may be added artificially. Example transmembrane domain sequences are eg. included in nt 1290-1421 of SEQ ID NO. 1, nt 2522-2611 of SEQ ID NO. 3 and nt 817-930 of SEQ ID NO. 4. These example sequences also comprise a cytoplasmic tail. The transmembrane domain may be localized 5' or 3' of the enzyme coding sequence.

The recombinant gene in the cell or construct may also comprise a promoter, which allows expression in the particular plant type. Example plant promoter may be used from any plant gene, e.g. from the GnTI, a fucosyltransferase, a xylosyltransferase or MnsI, in particular as provided herein according to nt 37-809 of SEQ ID NO. 1, nt 32-751 of SEQ ID NO. 2, nt 33-622 of SEQ ID NO. 3 or nt 37-536 of SEQ ID NO. 4. Any one of these can be operatively linked to any one of the coding sequences.

The coding sequences encoding the (heterologous) enzymes and the glycosylated proteins as described herein contain at least one type of promoter that is operable in a plant cell, for example, an inducible or a constitutive promoter operatively linked to a enzyme encoding nucleic acid sequence as herein defined and as provided by the present invention. This enables control of expression of the genes.

The term "inducible" as applied to a promoter is well understood by those skilled in the art. In essence, expression under the control of an inducible promoter is "switched on" or increased in response to an applied stimulus (which may be generated within a cell or provided exogenously). The nature of the stimulus varies between promoters. Some inducible promoters cause little or undetectable levels of expression (or no expression) in the absence of the appropriate stimulus. Other inducible promoters cause detectable constitutive expression in the absence of the stimulus. Whatever the level of expression is in the absence of the stimulus, expression from any inducible promoter is increased in the presence of the correct stimulus. Upon application of the stimulus, expression is increased (or switched on) to a level, which brings about the desired phenotype.

Before use in the inventive plant cell, the constructs of the invention can be amplified as is known in the art, e.g. in a bacterium such as *E*. *coli.* The construct may be incorporated in an amplification vector. The vector can have suitable elements for bacterial amplification such as an origin of replication or other elements suitable for molecular cloning. The construct may have a cassette with the genetic elements suitable for genomic integration of the transgenes flanked by restriction sites. Bacterial amplification elements are usually outside of this cassette.

In preferred embodiments, the construct elements for plant genome integration of the inventive transgenes can be removed from such an amplification vector by a restriction enzyme acting on the restriction sites. The construct can then be used for the transformation of the plant cells.

The inventive transgenes and coding sequences can be included in a cassette for particular genetic localization and integration. Specific genes or other genetic portions (e.g. noncoding) may be targeted leading to specific hybridization and integration of the recombinant sequences. To this end, the inventive coding sequences may be flanked by genetic localization sequences, preferably wherein said genetic localization sequences target a plant gene selected from GlcNAc-transferase I (GnTI), mannosidase I (MnsI), alpha-1,3-fucosyltransferase or beta-1,2-xylosyltransferase. In particular preferred embodiments these genes are inactivated, as mentioned above, preferably by knock-out. With these localizations of the recombinant sequences it is possible to introduce the inventive heterologous sequences and remove one of the unwanted sequences at the same time. In particular, the present invention also provides a plant cell, wherein integration of one of the recombinant sequences in any one of these plant gene sequences has occurred, but of course also other integration sites are possible.

For localization, it is e.g. possible to use coding sequences of the target gene or 5' UTR and/or 3' UTR sequences of these genes. Such sequences are e.g. provided in SEQ ID NO. 1-4 as shown in the example section.

Also provided is a kit comprising genetic constructs for GNPT and NAGPA, wherein said genetic constructs are in separate or a linked nucleic acid molecule(s). These constructs are preferably employed in combination as explained above. The constructs may e.g. be in separate containers such as vials. In any embodiment or aspect, constructs for GNPT may include separate constructs for GNPTAB and GNPTG.

The present invention is further illustrated by the following figures and examples without being limited to these embodiments of the present invention.

### Figures:

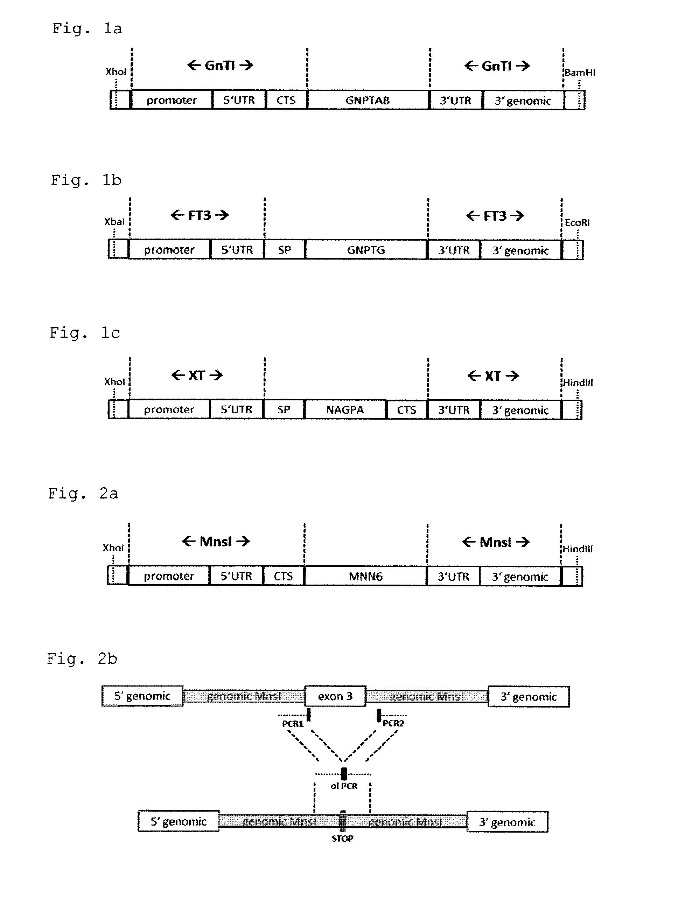

**Fig 1a** shows a construct for homologous recombination of human GNPTAB cDNA into the GnTI-locus from *Physcomitrella.* This construct comprises homologous sequences **(← GnTI →)** of the *Physcomitrella* GnTI promoter **(Pro)** and 5'UTR on the 5' end and homologous sequences of the GnTI 3'UTR and adjacent genomic region on the 3' end. The homologous sequences target the human GNPTAB cDNA **(GNPTA, GNPTB)** to the GnTI locus and replace the endogenous GnTI by homologous recombination. This provides the possibility to generate loss-of-function mutants for GnTI simultaneously. To obtain expression of GNPTAB even without homologous recombination, the GnTI promoter in this construct is functional and provides independent expression of GNPTAB in case of illegitimate recombination. Correct localization of the expressed GNPT to the cis-Golgi is achieved by replacing the CTS-domain of the alpha subunit of GNPTAB by the CTS-domain of GnTI from *Physcomitrella* **(CTS)** in this construct. All constructs are transformed into *Physcomitrella* protoplasts as linear DNA. Possible restriction enzymes for DNA-linearization are XhoI and BamHI.
**Fig 1b** shows a construct for homologous recombination of human GNPTG cDNA into the FT³-locus from *Physcomitrella.* This construct comprises homologous sequences **(← FT3 →)** of the *Physcomitrella* FT³ promoter and 5'UTR on the 5' end and homologous sequences of the FT³ 3'UTR and adjacent genomic region on the 3' end. The homologous sequences should target the human GNPTG cDNA **(GNPTG)** to the FT³ locus and replace the endogenous FT³ by homologous recombination. In addition, the FT³ promoter in this construct is functional and provides independent expression of GNPTG in case of illegitimate recombination. Using a plant signal peptide **(SP)** in this construct ensures gamma subunit of GNPT entering the secretory pathway and gaining the possibility to interact with alpha and beta subunits, anchored in the Golgi membrane. All constructs are transformed into *Physcomitrella* protoplasts as linear DNA. Possible restriction enzymes for DNA-linearization are XbaI and EcoRI.
**Fig 1c** shows a construct for homologous recombination of human NAGPA cDNA into the XT-locus from *Physcomitrella.* This construct comprises homologous sequences **(← XT →)** of the *Physcomitrella* XT promoter and 5'UTR on the 5' end and homologous sequences of the XT 3'UTR and adjacent genomic region on the 3' end. The homologous sequences should target the human NAGPA cDNA **(NAGPA)** to the XT locus and replace the endogenous XT by homologous recombination. In addition, the XT promoter in this construct is functional and provides independent expression of NAGPA in case of illegitimate recombination. Using a plant signal peptide **(SP)** in this construct and the C-terminal CTS-domain of a *trans*-Golgi-localized *Physcomitrella* protein **(CTS)** replacing the NAGPA CTS-domain ensures correct targeting of expressed NAGPA to the *trans*-Golgi in moss cells. All constructs are transformed into *Physcomitrella* protoplasts as linear DNA. Possible restriction enzymes for DNA-linearization are XhoI and HindIII.
**Fig 2a** shows a construct for homologous recombination of yeast Mnn6 cDNA into the MnsI-locus from *Physcomitrella.* This construct comprises homologous sequences **(← MnsI →)** of the *Physcomitrella* mannosyl-oligosaccharide alpha-1,2-mannosidase I (MnsI) promoter and 5'UTR on the 5' end and homologous sequences of the MnsI 3'UTR and adjacent genomic region on the 3' end. The homologous sequences target the yeast Mnn6 cDNA to the MnsI locus and replace the endogenous MnsI by homologous recombination. This provides the possibility to generate knockout mutants for MnsI simultaneously. In addition, the MnsI promoter in this construct is functional and provides independent expression of Mnn6p in case of illegitimate recombination. Targeting of the expressed Mnn6p to the ER/ cis-Golgi is achieved by replacing the Mnn6 CTS-domain by the native *Physcomitrella* MnsI CTS-domain. All constructs are transformed into *Physcomitrella* protoplasts as linear DNA. Possible restriction enzymes for DNA-linearization are XhoI and HindIII.
**Fig 2b** shows a PCR-generated construct for targeted knock-out of MnsI genes by homologous recombination. Two PCR-products are generated, amplifying genomic regions of MnsI-locus adjacent to an important exon (e.g. exon 3) and adding overlapping ends including a stop-codon (black squares). In a third, overlapping PCR (ol PCR) the final construct is amplified, which is transformed into moss protoplasts, replacing the original sequence after homologous recombination. The final sequence is depleted of an important part and harbors an in-frame stop-codon (black square) instead.

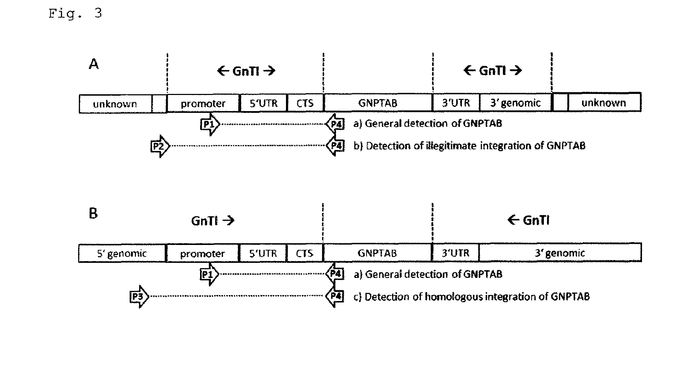

**Fig 3** shows a PCR-based identification of construct integration: GNPTAB cDNA targeting the GnTI-locus of *Physcomitrella* **(A)** In case of illegitimate integration, GNPTAB construct is detectable by PCR using GNPTAB-specific primer P4 and construct-specific primer P1 (case a) or primer P2, which binds to the non-homologous extension of the construct (case b). **(B)** In case of homologous integration, GNPTAB construct is detectable by PCR using GNPTAB-specific primer P4 and construct-specific primer P1 (case a) or primer P3, which binds to the 5' adjacent genomic region of GnTI-locus (case c).

### Examples:

### Abbreviations

CTS = Cytoplasmic tail, transmembrane domain and stem region
ER = Endoplasmic reticulum
FT³ = Alpha-1,3-fucosyltransferase (This enzyme transfers fucose to the core structure of N-glycan chains with an alpha 1,3-linkage. This modification is plant specific and has potential to trigger immune responses in mammals.)
GNPT = N-acetylglucosamine-1-phosphotransferase (In the Golgi apparatus, the heterohexameric complex (two alpha and beta subunits encoded by the gene GNPTAB and two gamma subunits encoded by the gene GNPTG) catalyzes the first step in the synthesis of mannose 6-phosphate recognition markers on certain oligosaccharides of newly synthesized lysosomal enzymes. The enzyme adds a GlcNAc-phosphate residue to an N-glycan mannose.)
GnTI = GlcNAc-transferase I (This enzyme transfers GlcNAc residues to 1,3-linked mannose on N-glycan chains after the removal of the 1,2-linked mannoses by mannosidases.)
NAGPA = N-acetylglucosamine-1-phosphodiester alpha-N-acetylglucosaminidase or "uncovering enzyme" (This enzyme functions as a homotetramer of two disulfide-linked homodimers to remove the GlcNAc residues added formerly by GNPT leaving the phosphate on the N-glycan mannose uncovered. In addition to having an N-terminal signal peptide, the protein's C-terminus contains multiple signals for trafficking it between lysosomes, the plasma membrane, and trans-Golgi network.)
MnsI = Mannosyl-oligosaccharide alpha-1,2-mannosidase I (These enzymes remove 1,2-linked mannose from N-glycan chains during trimming in the ER and early Golgi, reducing Man₉-structures up to Man₅-structures. Mannosidases from class II then further reduce the glycan-chains to Man₃-structures by removing one 1,3-linked and one 1,6-linked mannose.)
Mnn4p, encoded by the gene Mnn4 (This is a regulatory protein from *Saccharomyces cerevisiae* that enhances Mnn6p activity, probably by replacing a negative regulator binding to Mnn6p. The complete function is not totally understood.)
Mnn6p = Mannosyl-phosphate transferase Mnn6p, encoded by the gene Mnn6 (This transferase from *Saccharomyces cerevisiae* adds mannosyl-phosphate moieties to alpha-1,2-linked mannose on glycan structures.)
ORF = open reading frame
PEG = Polyethylene glycol
Rpm = Rotations per minute
SP = Signal peptide
UTR = un-translated region
XT = Beta-1,2-xylosyltransferase (This enzyme transfers xylose to the core structure of N-glycan chains with a beta 1,2-linkage. This modification is plant specific and has potential to activate immune response in mammals.)

### Example 1: Outline of experiments:

*Physcomitrella patens* moss strains were created expressing recombinant human alpha-galactosidase as a basis to demonstrate mannose-6-phosphorylation in plants. Additionally, plant-specific xylose- and fucose-addition to N-glycan cores was blocked in this strain, due to loss of beta-1,2-xylosyltransferase and alpha-1,3-fucosyltransferase (as in WO 2004/057002). Different additional enzymes were expressed to achieve mannose-6-phosphorylation. Except the constructs depicted in **Fig.2b****,** all DNA constructs **(****Fig.1a****-c and 2a)** were synthesized and cloned into plasmids at GeneArt (GeneArt/ Life Technologies) and amplified in *E. coli* DH5 alpha strains (NEB). The amplified DNA was subsequently cut (using the both depicted restriction enzymes), excised from an agarose gel and purified using gel extraction kits (Thermo Scientific).

### Example 2: Recombinant expression of GNPT and NAGPA

The human enzymes GNPT and NAGPA were co-expressed in a moss strain producing recombinant human alpha-galactosidase as an example of a glycoprotein. Any glycoprotein could be used. Therefore, three different expression constructs were used as shown in **Fig.1a****-c.** Homologous integration of these gene constructs was intended to target transcriptionally active loci, with as few interruptions of the genetic background as possible (like the already mutated XT and FT³ loci). Nevertheless, the GNPTAB-construct was used to target GnTI-locus to simultaneously create a loss-of-function mutant of GnTI. GnTI normally transfers GlcNAc residues onto mannose as the first step in complex-type glycan biosynthesis. Lack of this specific GnTI activity blocks complex glycan formation, stopping processing of the glycan chains at a high-mannose state (mainly Man5), which should act as substrates for mannose-6-phosphorylation by GNPT. To obtain expression of the enzymes even without homologous recombination, the promoters used in the constructs were functional and provide independent expression in case of illegitimate recombination. Correct localization of the human enzymes to the plant Golgi was achieved by replacing the CTS-domain (includes the signal peptide) of the alpha subunit of GNPTAB and NAGPA by the CTS-domain of GnTI (includes the signal peptide) and glycosyl hydrolase family 17 protein (NCBI Phypadraft_172885, XP_001784088.1) from *Physcomitrella,* respectively. In addition, using a plant signal peptide (SP) ensures gamma subunit of GNPT and NAGPA entering the plant secretory pathway.

### Sequences:

SEQ ID NO. 1 provides the GNPTAB expressing and GnTI targeting construct (Fig. 1a) with the following elements:
   Nucleotides 1-36: Non-homologous extension
   Nucleotides 37-809: GnTI promoter
   Nucleotides 810-1289: GnTI 5'UTR
   Nucleotides 1290-1421: GnTI CTS
   Nucleotides 1422-5060: GNPTAB cDNA
   Nucleotides 5061-5661: GnTI 3'UTR
   Nucleotides 5861-5896: Non-homologous extension
   The coding region spans nucleotides 1290-5057 (starting with the GnTI CTS start codon and ending before the GNPTAB stop codon).
SEQ ID NO. 2 provides the GNPTG expressing and FT³ targeting construct (Fig. 1b) with the following elements:
   Nucleotides 1-31: Non-homologous extension
   Nucleotides 32-751: FT³ promoter
   Nucleotides 752-1093: FT³ 5'UTR
   Nucleotides 1094-1171: Plant signal peptide
   Nucleotides 1172-2017: GNPTG cDNA
   Nucleotides 2018-2135: FT³ 3'UTR
   Nucleotides 2718-2748: Non-homologous extension
      The coding region spans nucleotides 1094-2014 (starting with the signal peptide start codon and ending before the GNPTG stop codon).
SEQ ID NO. 3 provides the NAGPA expressing and XT targeting construct (Fig. 1c) with the following elements:
   Nucleotides 1-32: Non-homologous extension
   Nucleotides 33-622: XT promoter
   Nucleotides 623-1198: XT 5'UTR
   Nucleotides 1199-1276: Plant signal peptide
   Nucleotides 1277-2521: NAGPA cDNA coding sequence
   Nucleotides 2522-2611: Glycosyl hydrolase family 17 protein CTS
   Nucleotides 2612-2930: XT 3'UTR
   Nucleotides 3262-3293: Non-homologous extension
   The coding region spans nucleotides 1199-2608 (starting with the signal peptide start codon, spanning the NAGPA cDNA coding sequence and ending before the CTS stop codon).

### Example 3: Recombinant expression of Mnn6p

In the second strategy, the yeast enzyme Mnn6p was expressed in a moss strain producing human alpha-galactosidase as an example of a glycoprotein. Any glycoprotein could be used. To generate stable transgenic moss cell lines, one main expression construct was used **(****Fig.2a****).** Homologous integration of this gene construct was targeting a *Physcomitrella* MnsI to simultaneously create a loss-of-function mutant for this MnsI. Abolishing MnsI activity in the cells leads to accumulation of high-mannose N-glycan structures harboring the important 1,2-linked mannoses as acceptors for mannosyl-phosphate transferred by Mnn6p. To obtain expression of the enzyme even without homologous recombination, the promoter used in this construct was functional and provides independent expression in case of illegitimate recombination. Correct localization of the yeast enzyme to the plant ER and Golgi was achieved by replacing the CTS-domain (includes the signal peptide) of the Mnn6p by the CTS-domain of MnsI (includes the signal peptide) from *Physcomitrella.*

Additional homologues of MnsI (four MnsI genes are present in *Physcomitrella*) can be targeted by separate constructs to generate a multiple MnsI loss-of-function mutant. These constructs (see example in **Fig.2b****)** are generated by overlapping PCR, thereby deleting important parts (exon) of the coding sequence and adding a stop-codon to the ORF. The amplified homologous sequences were subsequently used for recombination into the original locus, replacing the coding sequence by the truncated PCR-construct. MnsI-mutants exhibit a further increase in high-mannose N-glycan structures harboring the important 1,2-linked mannoses as acceptors for mannosyl-phosphate transferred by Mnn6p.

### Sequences:

SEQ ID NO. 4 provides the Mnn6 expressing and MnsI targeting construct (Fig. 2a) with the following elements:
   Nucleotides 1-36: Non-homologous extension
   Nucleotides 37-536: MnsI promoter
   Nucleotides 537-816: MnsI 5'UTR
   Nucleotides 817-930: MnsI CTS
   Nucleotides 931-2172: Mnn6 cDNA
   Nucleotides 2173-2662: MnsI 3'UTR
   Nucleotides 2923-2958: Non-homologous extension
   The coding region spans nucleotides 817-2169 (starting with the MnsI CTS start codon and ending before the Mnn6 stop codon).

### Example 4: Transformation of moss protoplasts

Linearized and purified DNA-constructs were precipitated under sterile conditions, adjusted with sterile water to a concentration of 0.4 µg/µl and used to transform *Physcomitrella* protoplasts generated from protonemal cell cultures. Cultivation of moss cultures was performed as described by Reski, R. and Abel, W. (1985) Planta, 165, 354-358. Preparation of protoplasts and transformation (PEG-based) was performed as described by Strepp et al. (1998) Proc. Natl. Acad. Sci. USA, 95, 4368-4373.

### Example 5: PCR-screening of transformed moss strains

Obtained moss colonies were screened for genomic integration of the constructs by PCR. In addition, specific primers **(Tab. 1)** were used in two further PCRs to discriminate between homologous and illegitimate integration events (see example in **Fig.3****).** Therefore, genomic DNA was extracted in a 96-well format. In brief, moss material was picked with a forceps, transferred into a deep-well tube for 96-well plates together with a glass bead and frozen in liquid nitrogen. The material was ground in a cold mixer mill (Retch Mixer Mill) for 2 minutes with a frequency of 30 rpm and the cold samples were centrifuged quickly at 4600 rpm. Extraction buffer (250 µl) was added to each sample and the plate was mixed for 10 seconds on a vortex. Plates were centrifuged for 35 minutes at 4600 rpm and 4°C and supernatant (180 µl) was transferred to new wells containing 180 µl isopropyl alcohol. Samples were incubated for 20 minutes on ice and centrifuged for 15 minutes at 4600 rpm and 4°C. The supernatant was discarded and DNA pellets were dried shortly in air. Subsequently, DNA was resolved in 50 µl DNase-free water and used in a PCR-based screening **(****Fig.3****).**

### Sequences:

| # | Sequence | Purpose |
|---|---|---|
| P1 | GCCAATGCACAAGTTTGACATC (SEQ ID NO. 5) | General detection of integrated GNPTAB together with P4 |
| P2 | CCACTCCTATGCCAATAGTGACTC (SEQ ID NO. 6) | Binds 5' non-homologous extension in the constructs 1a and 2; Detection of heterologous integration of GNPTAB together with P4 or Mnn6 together with P15 |
| P3 | GTAGCCAAAAAACTTAGCCAAC (SEQ ID NO. 7) | Binds 5' genomic region of GnTI; Detection of homologous integration of GNPTAB together with P4 |
| P4 | CCCAGGTGTAAACAACGTCAATCG (SEQ ID NO. 8) | Detection of integrated GNPTAB |
| P5 | CATGCGAACGTGGCATAC (SEQ ID NO. 9) | General detection of integrated GNPTG together with P8 |
| P6 | CCGTCAGTCTTCCTCTTCC (SEQ ID NO. 10) | Binds 5' non-homologous extension in construct 1b; Detection of heterologous integration of GNPTG together with P8 |
| P7 | AGTAAGATGGAGTCACATTAG (SEQ ID NO. 11) | Binds 5' genomic region of FT³; De-tection of homologous integration of GNPTG together with P8 |
| P8 | CTCCTCCACCACCTTCATC (SEQ ID NO. 12) | Detection of integrated GNPTG |
| P9 | GTCGGTTGTCAGTTGGATG (SEQ ID NO. 13) | General detection of integrated NAGPA together with P12 |
| P10 | CGCCAAGAACTCACTGTATC (SEQ ID NO. 14) | Binds 5' non-homologous extension in construct 1c; Detection of heterologous integration of NAGPA together with P12 |
| P11 | GGGAAGAAGAGAAGCAGAGAAG (SEQ ID NO. 15) | Binds 5' genomic region of XT; De-tection of homologous integration of NAGPA together with P12 |
| P12 | ATAGGGCAGTAGCAAGTCG (SEQ ID NO. 16) | Detection of integrated NAGPA |
| P13 | TGGCATAAGTCGTCCTTC (SEQ ID NO. 17) | General detection of integrated Mnn6 together with P15 |
| P14 | GATGCTTCAGCCATCTAACC (SEQ ID NO. 18) | Binds 5' genomic region of MnsI; Detection of homologous integration of Mnn6 together with P15 |
| P15 | CCTAGCGTCGTTTCATTACC (SEQ ID NO. 19) | Detection of integrated Mnn6 |

### Example 6: Analysis of N-glycan structure on proteins of transgenic moss strains

Identified moss strains, which integrated the transgenic constructs were cultivated as protonemal cultures for 4 weeks in culture flasks, with weekly sub-culturing. Subsequently, moss material was harvested, frozen in liquid nitrogen and stored at -80°C.

The analysis of the glycan structures was carried out, with some modifications, as described in Koprivova et al. 2004 (Plant Biotechnology Journal (2004) 2, pp. 517-523. In brief, a portion of 300 - 500 mg of frozen moss tissue was homogenized in 2.5 ml of 0.1 M TRIS-Buffer of pH 8.0 with an Ultrathurrax. The slurry was digested overnight with 0.5 mg thermolysin at 70°C. Glycopeptides were isolated from the digest by binding to Dowex 50W2 equilibrated with 2 % acetic acid. The bound glycopeptides and peptides were subsequently eluted with 0.5 M ammonium acetate with pH 6.0. The orcinol-test positive fractions were combined, the volume was reduced using a rotary evaporator and the glycopeptides were then purified by gel filtration over Sephadex G25 fine in 1 % acetic acid. The high molecular mass orcinol-positive fractions were pooled and dried in a Speed-Vac. Glycopeptides were then digested with peptide:N-glycosidase A (pro-glycan, Austria) in 0.1 M ammonium acetate with pH 5.0. The resultant oligosaccharides were isolated by a second passage over Dowex 50W2, whereby now the nonbinding fraction was collected and further purified by passage over a 25 mg Zorbax SPE C18 cartridge (Agilent, Waldbronn, Germany). The aqueous flow-through was lyophilized, dissolved in 20 µL of water and subjected to MALDI mass spectrometry on an Autoflex MALDI-TOF MS (Bruker Daltonics, Bremen, Germany) using 2,5-dihydroxybenzoic acid (2% in 30% acetonitrile) as the matrix. Mono-isotopic sodium adduct ions were thereby measured with a mass accuracy of usually better than 0.2 Da.

Mannose-6-phosphate modified alpha-galactosidase could be detected thus providing evidence that the recombinant expression approach in moss was successful.

## Claims

1. The method of expressing a glycoprotein with a mammalian-type phosphoglycosylation in a plant cell comprising expressing said glycoprotein in a plant cell, wherein said plant cell recombinantly expresses
i) N-acetylglucosamine-1-phosphotransferase (GNPT) and N-acetylglucosamine-1-phosphodiester-alpha-N-acetylglucosaminidase (NAGPA), or
ii) mannosyl-phosphate-transferase Mnn6p.

2. A plant cell comprising recombinant genes for one or more enzymes selected from
i) N-acetylglucosamine-1-phosphotransferase (GNPT) and N-acetylglucosamine-1-phosphodiester-alpha-N-acetylglucosaminidase (NAGPA), or
ii) mannosyl-phosphate-transferase Mnn6p.

3. The method or plant cell of claim 1 or 2, wherein said plant cell further has a reduced activity, preferably a complete loss of function, of GlcNAc-transferase I (GnTI), in particular by knock-out, especially preferred by interrupting the GnTI gene of said plant, even more preferred by a gene of any one of the recombinantly expressed proteins or a separately expressed subunit thereof, such as GNPT-alpha, -beta and/or -gamma.

4. The method or plant cell of any one of claims 1 to 3, wherein said plant cell further has a reduced activity, preferably a complete loss of function, of mannosidase I (MnsI), in particular by knock-out mutation of an endogenous MnsI gene, especially preferred by interrupting the MnsI gene of said plant cell, even more preferred by a gene of any one of the recombinantly expressed proteins, or by administering a mannosidase inhibitor to said plant cell.

5. The method or plant cell of any one of claims 1 to 4, wherein said plant cell further has a reduced activity, preferably a complete loss of function, of alpha-1,3-fucosyltransferase and/or beta-1,2-xylosyltransferase, in particular by knock-out, especially preferred by interrupting the alpha-1,3-fucosyltransferase and/or beta-1,2-xylosyltransferase encoding gene of said plant, preferably by a gene of any one of the recombinantly expressed proteins.

6. The method of any one of claims 1 to 5, wherein said plant cell is of a plant selected from bryophyta, preferably moss, especially preferred of the class bryopsida, such as *Physcomitrella patens,* or a liverwort, hornwort, or aquatic plant, preferably of the genus *Lemna, Spirodela, Landoltia, Wolffia* or *Wolffiella.*

7. The method or plant cell of any one of claims 1 to 6, wherein GNPT is expressed by recombinant GNPTAB and GNPTG genes.

8. The method or plant cell of any one of claims 1 to 7, wherein said plant cell is an isolated cell, a singularized cell, a cell in or of a plant tissue, preferably a tissue selected from callus, protonema, phloem, xylem, mesophyll, trunk, leaves, thallus, chloronema, caulonema, rhizoid or gametophore, or a cell in a plant organism.

9. The method of any one of claims 1 to 8, wherein said glycoprotein is selected from alpha-galactosidase, acid alpha-glucosidase, beta-hexosaminidase A, arylsulfatase A, arylsulfatase B, lysosomal alpha-mannosidase, L-asparaginase, lysosomal acid lipase, acid ceramidase, beta-galactosidase, alpha-L-fucosidase, alpha-L-iduronidase, iduronate sulfatase, N-acetylglucosamine 6-sulfatase, beta-glucuronidase, Sphingomyelin phosphodiesterase, alpha-N-acetylgalactosaminidase, palmitoyl-protein thioesterase, tripeptidyl-peptidase, ceroid-lipofuscinosis neuronal protein 5, cathepsin D.

10. The method of any one of claims 1 to 9 further comprising the step of removing a terminal (non-reducing end) sugar residue, preferably mannose, lacking a 6-phosphate, preferably by treating the expressed glycoprotein with a glycosidase.

11. A genetic expression construct comprising a coding sequence of an enzyme selected from
a) N-acetylglucosamine-1-phosphotransferase (GNPT) or any one of its subunits,
b) N-acetylglucosamine-1-phosphodiester-alpha-N-acetylglucosaminidase (NAGPA), and
c) mannosyl-phosphate-transferase Mnn6p,
wherein said construct comprises a plant golgi signal sequence operatively linked to said enzyme coding sequence and/or a transmembrane sequence as part of said coding sequence operatively linked to said enzyme coding sequence, and a plant promoter sequence.

12. The genetic construct according to claim 11, wherein a non-plant signal sequence of the enzyme is removed.

13. The genetic construct according to claim 11 or 12, wherein said coding sequence is flanked by genetic localization sequences, preferably wherein said genetic localization sequences targets a plant gene selected from GlcNAc-transferase I (GnTI), mannosidase I (MnsI), alpha-1,3-fucosyltransferase or beta-1,2-xylosyltransferase.

14. A kit comprising genetic constructs according to any one of claims 11 to 13 for GNPT and NAGPA, wherein said genetic constructs are in separate or a linked nucleic acid molecule(s).

15. Method of producing the plant cell according to any one of claims 2 to 8 comprising transforming said plant cell by introducing one or more constructs, optionally in combination, as set forth in claims 11 to 14 into a plant cell.
